# FASCICULE DE BREVET EUROPEEN

(11) **EP 1 415 214 B1**
(45) Date de publication et mention de la délivrance du brevet: **22.06.2005**
(21) Numéro de dépôt: 02774823.5
(22) Date de dépôt: 23.07.2002
(51) Int. Cl.: G06F 1/00, G06F 17/00

(54) **EQUIPEMENTS DESTINES A L'ANALYSE DE SIGNAUX BIOLOGIQUES REPRESENTATIFS DES VARIATIONS DE LA PRESSION INTRACRANIENNE ET DE LA PRESSION SANGUINE**
SYSTEM ZUR ANALYSE VON BIOLOGISCHEN SIGNALDATEN DES INTRAKRANIELLEN DRUCKS UND DES BLUTDRUCKES
EQUIPMENT FOR ANALYSING BIOLOGICAL SIGNALS REPRESENTING INTRACRANIAL PRESSURE AND BLOOD PRESSURE VARIATIONS

(30) Priorité: 23.07.2001 FR 0109807
(43) Date de publication de la demande: 06.05.2004
(73) Titulaire: Université d'Auvergne, 63001 Clermont-Ferrand Cedex 1 (FR)
(72) Inventeur: LEMAIRE, Jean-Jacques, F-63122 Saint Genes Champanelle (FR)
(74) Mandataire: Breese, Pierre
(86) Numéro de dépôt international: PCT/FR2002/002632
(87) Numéro de publication internationale: WO 2003/010617

(56) Documents cités:
- WO-A-01/37724
- US-A- 4 893 630
- HARA K ET AL.: "DETECTION OF THE B WAVES IN THE OSCILLATION OF INTRACRANIAL PRESSURE BY FAST FOURIER TRANSFORM" MED. INFORM., vol. 15, no. 2, 1990, pages 125-131, XP008001771
- LEMAIRE J J ET AL: "A computer software for frequential analysis of slow intracranial pressure waves" COMPUTER METHODS AND PROGRAMS IN BIOMEDICINE, 1 FEB. 1994, NETHERLANDS, vol. 42, no. 1, pages 1-14, XP008001796 ISSN: 0169-2607 cité dans la demande
- AKAY M: "SPATIAL MAPPING OF RESPIRATORY RELATED EVOKED RESPONSES USING WAVELET TRANSFORM METHOD" PROC. OF THE FIRST JOINT BMSE/EMBS CONFERENCE, 13 octobre 1999 (1999-10-13), XP010357853 ATLANTA, GA, USA
- MEYER Y ET AL: "L'ANALYSE PAR ONDELETTES" POUR LA SCIENCE, EUGENE BELIN, PARIS, FR, 1 septembre 1987 (1987-09-01), pages 28-37, XP002008004 ISSN: 0153-4092

## Description

La présente invention se rapporte au domaine des équipements destinés à l'analyse de signaux biologiques représentatifs des variations de la pression intracrânienne et de la pression sanguine, et pour cette dernière notamment dans le crâne.

Le but de telles analyses est d'aider le clinicien dans l'interprétation des données fournies par des capteurs fournissant des signaux représentatifs de la pression intracrânienne (ICP - intracranial Pressure), et des signaux liés : la pression sanguine , la vitesse de circulation artérielle ou veineuse, et les concentrations gazeuses. Elles permettent au clinicien d'en déduire les traitements adaptés à la pathologie déduite de ces informations. Ces analyses ont fait l'objet de différents travaux scientifiques, par exemple :
* Lemaire et al, "a computer software for fréquentiel analysis of slow intracranial pressure waves", Comput. Methods Programs Biomed, 1984, 42, 1-14,
* Daley et al, "Fluctuation of Intracranial Press. Assoc with the Cardiac Cycle", Journal of Neurosurgery, vol. 11, No. 5, 11-1982, pp. 617-621,
* Avezaat et al; "Cerebrospinal Fluid Pulse Press and Intracranial vol.-Press Relationships", J. Neurol., Neursurg, and Psych., 1979, 42, pp. 687-700,
* Portnoy et al.; "Cerebrospinal Fluid Pulse Wave as an Indicator of Cerebral Nutoreg."; J. Neurosurg., vol. 56, 5-1982-pp. 666-678,

Pour réaliser de telles analyses, on a proposé dans l'état de la technique des équipements pour l'acquisition et le traitement des signaux de pression. WO132076 A décrit par exemple un appareil de surveillance permettant de déterminer un paramètre physiologique chez un patient. Cet appareil comporte un dispositif d'étalonnage configuré pour fournir un signal d'étalonnage représentatif du paramètre physiologique. On place un capteur non invasif sur le vaisseau, ce capteur non invasif étant configuré pour détecter un paramètre sanguin et pour produire un signal représentatif du paramètre sanguin. On définit, dans ce contexte, un paramètre sanguin tel que la tension, le débit, le volume, la vitesse, le mouvement et la position de la paroi du vaisseau et d'autres paramètres apparentés. Un processeur est configuré pour déterminer la relation existant entre une caractéristique de l'onde d'excitateur reçue et une caractéristique du paramètre physiologique.

WO9849934 A décrit un appareil et un procédé non invasif de mesure de la pression intracrânienne. Le système de mesure émet des signaux acoustiques traversant le crâne au moyen des émetteurs et fournit une indication de la pression intracrânienne fonction du signal acoustique reçu après interaction avec le cerveau. Des propriétés telles que l'impédance de la transmission acoustique, la fréquence de résonance, les caractéristiques de résonance, la vitesse de son, et autres peuvent se mesurer et être corrélées avec la tension intracrânienne. Les signaux acoustiques présentent par exemple des fréquences caractéristiques de moins de 100 kHz, dans les plages audibles et infrasonores. L'intensité du signal acoustique servant à mesurer la tension intracrânienne est relativement faible d'où peu ou pas de dangers pour la santé lors d'examens courts ou longs.

WO068647 A concerne une méthode permettant de surveiller de manière non invasive la pression intracrânienne d'un patient. On obtient au moins un oscillogramme représentant une pulsation d'une caractéristique anatomique de la tête du patient, de préférence en intégrant des traces de réflexion d'ultrasons dans une porte temporelle correspondant à des réflexions de ladite caractéristique. De préférence, ladite caractéristique anatomique est le troisième ventricule cérébral. On infère une mesure quantitative de la pression intracrânienne à partir d'au moins deux caractéristiques de diagnostic, telles que des heures de diagnostic, associées à l'oscillogramme. Dans une variante, on obtient une mesure qualitative de la pression intracrânienne à partir de la forme de la courbe respiratoire imposée au train d'ondes par la respiration du patient.

WO9926529 A décrit une unité de traitement à transformation de Fourier rapide appliquée à une analyse de fréquence à une forme d'onde (MHj) sans mouvement corporel et fournit des données d'analyse de forme d'onde (MKD). Parallèlement, une unité d'extraction d'onde descendante et une unité d'extraction d'onde liée à une incisure fournissent respectivement des données d'onde descendante (tide wave data, TWD) et des données d'onde dicrote liée à une incisure (dicrotic wave data, DWD), qui représentent respectivement une onde descendante et une onde dicrote liée à une incisure. Ensuite, une unité d'évaluation du pouls fournit des données relatives à l'état du pouls (ZD) sur la base des données TWD et DWD, moyennant quoi une unité de notification établit un rapport sur l'état du pouls du sujet considéré.

On connaît également le brevet américain US4893630 décrivant un équipement et un procédé pour l'analyse de la pression dans un organe vivant par un capteur délivrant un signal analogique, comprenant un convertisseur analogique-digital et une analyse par transformée de Fourier pour délivrer une distribution des fréquences du signal fournit par les capteurs de pression.

Ces différentes solutions ne sont pas totalement adaptées pour fournir des informations particulières utiles au clinicien.

L'invention vise à remédier à ce problème en proposant un système pour la représentation et l'analyse de variables de pression comportant des capteurs de pression [pression artérielle, pression intracardiaque], des moyens de traitement des signaux délivrés par lesdits capteurs et des moyens d'affichage des variations desdits signaux caractérisé en ce que les moyens de traitement comportent une pluralité d'entrées pour recevoir des signaux analogiques provenant de différents capteurs, chaque entrée étant raccordée à un circuit d'échantillonnage fournissant un signal numérique exploité par un calculateur pour réaliser des traitements comprenant :
- le ré-échantillonnage des signaux pour l'expansion du signal visualisé ou enregistré en blocs de N points , avec N étant puissance de deux,
- l'analyse de fréquence dudit signal échantillonné par rapport à une gamme de fréquences cibles, choisie parmi les ondes de type B, infra B et/ ou ultra B, enregistrées dans une mémoire, pour la visualisation et l'enregistrement des variations temporelles des signaux correspondant aux ondes lentes,
la détermination du décalage temporelle entre les signaux correspondant à deux entrées distinctes.

L'analyse fréquentielle pour extraire l'information relative aux ondes lentes constitue une amélioration essentielle des équipements de l'art antérieur, car elle offre au clinicien des possibilités d'interprétation nouvelles.

Selon une première variante, les moyens d'analyse fréquentielle sont constitués par un calculateur appliquant une transformée de Fourier Rapide (FFT).

Selon une deuxième variante, les moyens d'analyse fréquentielle sont constitués par un calculateur appliquant une analyse par ondelettes.

Avantageusement, ladite gamme de fréquences cibles enregistrées comprend des fréquences comprises entre 8.10⁻³ Hertz et 50.10⁻³ Hertz.

Selon un mode de réalisation préféré, le système comporte un circuit d'échantillonnage réalisant un échantillonnage de chacun des signaux d'entrée à une première fréquence, et un circuit de ré-échantillonnage à une fréquence d'échantillonnage -inférieure à la première fréquence, pour l'enregistrement des signaux horodatés dans une mémoire.

Selon une autre variante, il comporte un module d'acquisition et de traitement des signaux local et au moins un poste de monitorage distant relié audit module d'acquisition local par un réseau de télécommunication.

L'invention concerne également un procédé d'analyse de signaux biologiques de pression comprenant une étape d'échantillonnage caractérisé en ce qu'il comporte en outre une étape d'analyse fréquentielle par rapport à une gamme de fréquences cibles correspondant à des ondes lentes de type B et UB.

Selon une variante, le procédé comporte en outre une étape de détermination du décalage temporelle entre les signaux correspondant à deux entrées distinctes.

L'invention sera mieux comprise à la lecture de la description qui suit, se référant à un exemple non limitatif de réalisation, où :

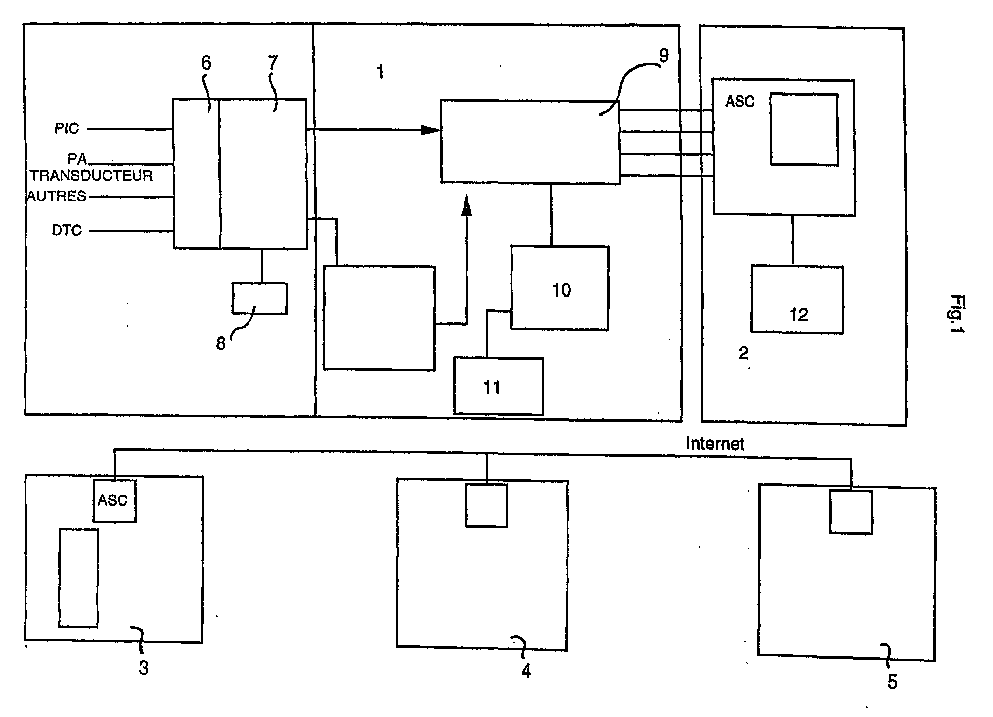

- la figure 1 représente une vue schématique d'un système selon l'invention ;
- la figure 2 représente une vue d'un écran d'affichage des informations visualisées par le système ;

L'équipement selon l'invention est composé de trois modules principaux :
- un module d'acquisition (1), généralement placé à proximité du patient
- un module de traitement (2), généralement placé chez le clinicien,
- un module d'exploitation et de visualisation qui peut être placé près du patient sous la forme d'un équipement (3), ou sous la forme d'un poste de travail local (4) relié au module de traitement par un réseau interne ou encore être constitué par un poste de travail distant (5) relié au module de traitement (2) par un réseau de télécommunication, par exemple Internet.

Le premier module (1) présente une pluralité de canaux d'entrée, pour recevoir des signaux provenant de différents capteurs de pression :
- capteur de pression artérielle (ABP - Arterial blood pressure)
- capteur de vitesse circulatoire cérébrale (CBF - cérébral blood pressure)
- capteur de pression intracranien (ICP - intracranial pressure).

Dans l'exemple décrit, le module (1) présente 8 canaux. Il comporte un circuit d'interface entrée-sortie (6) à 8 voies délivrant un signal de référence et des signaux analogiques correspondant aux différents canaux, sous forme parallèle ou multiplexée.

Les signaux analogiques sont ensuite échantillonnés par un circuit (7) commandé par une horloge (8). Chaque signal est échantillonné préférentiellement à 100 échantillons par secondes. Il peut être visualisé sur l'écran d'un moniteur (9) directement, ou faire l'objet d'une extraction d'un échantillon sur 8 pour être enregistré dans une mémoire (10) sous forme de séquences numériques horodatées, formant des tables associées à une information générale (nom du patient, nom du praticien,...). Eventuellement, le module comporte également des moyens de saisie d'une information libre sous forme de marqueur. Cette information est associée à l'information enregistrée, pour décrire par exemple un événement, ou pour annoter la courbe d'un des signaux observé.

Le module d'analyse (2) procède à une analyse fréquentielle des signaux échantillonnés à partir d'une fréquence cible choisie parmi :
- les ondes lentes de type « B », correspondant à une fréquence comprise entre 8 et 50 10⁻³ Hertz
- les ondes infra B correspondant à une fréquence inférieure à 8.10⁻³ Hertz
- les ondes ultra B correspondant à une fréquence comprise entre 50.10⁻³ Hertz et 200.10⁻³ Hertz.

Cette analyse s'effectue sur des blocs de N points (puissance de deux) échantillonnés, par exemple par blocs de 256 points. Le résultat est enregistré dans une mémoire (12). Une séquence échantillonnée d'un signal comprenant M échantillons se traduit donc par un fichier de M/N points correspondant à la variation de la transformée d'analyse fréquentielle.

L'analyse fréquentielle peut être réalisée par une transformée de Fourier Rapide (FFT).

Elle consiste à effectuer une analyse de fréquence spatiale à l'aide d'une transformée permettant de traduire une forme d'onde dans le domaine fréquentiel. Le résultat de la transformée est une suite de coefficients décrivant l'amplitude de chaque composante fréquentielle présente dans le bloc analysé.

L'analyse par FFT ou par Transformée Cosinus Discrète (DCT) caractérise chaque fréquence en multipliant le signal d'entrée par un exemple de la fréquence cible ou fonction de base, choisie parmi les fréquences des ondes lentes, et en intégrant le produit obtenu.

L'analyse est réalisée avec des circuits électroniques (DSP) connus, ou par l'application d'algorithmes connus, avec un taux d'échantillonnage (« samplingRate ») déterminé par le clinicien, par sélection de l'une des fréquences d'ondes lentes observées. Le paramètre N du nombre d'échantillon dans l'enregistrement analysé est prédéterminé, par exemple 256, ou variable par choix de l'utilisateur. Les algorithmes de transformée de Fourier discrète convertissent une fonction du temps à valeurs complexes échantillonnées en une fonction à valeurs complexes de la fréquence, également échantillonnée. Ils fournissent des informations telles que :
* Le tableau des coefficients des cosinus dans la formule de Fourier (partie réelle du résultat)
* Le tableau des coefficients des sinus dans la formule de Fourier (partie imaginaire du résultat).
* Le premier élément en sortie de chacun des tableaux contient la valeur moyenne de toutes les entrées. La variante de cet élément est affichée sur un moniteur, et fait l'objet de traitements comparatifs d'un signal à l'autre.

L'extraction des ondes lentes se fait à partir du spectre d'amplitude (et de puissance) et est réalisée par détection de la fréquence (F) dont l'amplitude (ou la puissance) est maximale (P) et ce pour les bandes de fréquences B, UB, et IB.

En particulier, le module d'analyse (2) comporte des moyens de calcul de décalage entre les ondes lentes de deux signaux, et de représentation sur l'afficheur d'un moniteur de ces décalages.

Cette analyse est par application d'une fonction de cohérence dans le domaine fréquentiel ou d'un coefficient de Pearson en temporel.

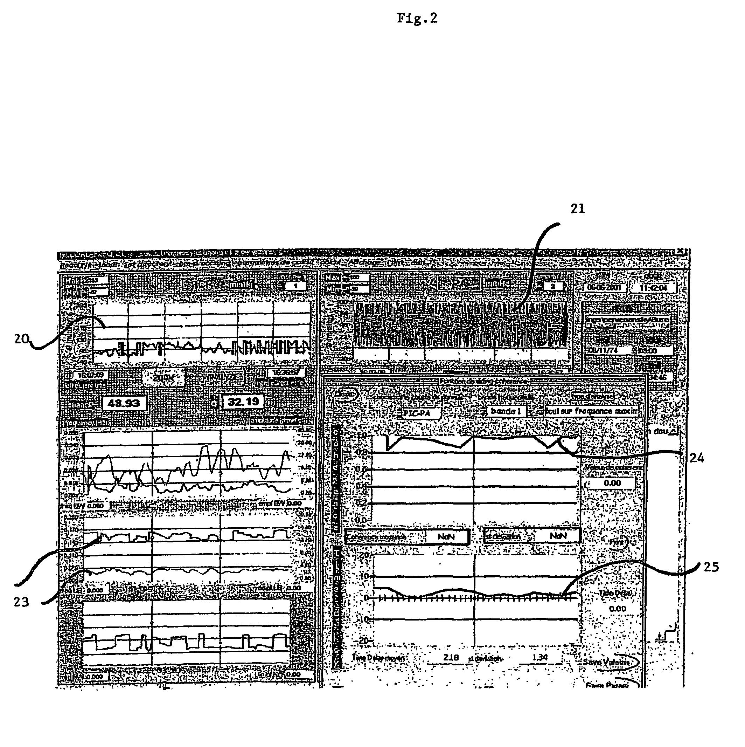

A titre d'exemple, la figure 2 représente la vue d'un écran d'affichage provenant d'un système selon l'invention.

L'écran est subdivisé en une pluralité de zones d'affichages pour la représentation :
- de la variation temporelle de la pression intracrânienne (zone (20))
- de la variation temporelle de la pression artérielle (zone (21))
- de la variation de la composante correspondant à l'onde lente B, avec une courbe (22) correspondant à la fréquence en millihertz et une courbe (23) correspondant à l'amplitude de la pression
- du taux de corrélation entre les signaux de pression intracrânienne (ou de la vitesse circulatoire (20) et de pression artérielle (21), sous la forme de la courbe (24)
- du décalage temporel entre les ondes lentes des signaux de pression intracrânienne ou de vitesse circulatoire ? (20) et de pression artérielle (21), sous la forme de la courbe (25)

## Revendications

1. Système pour la représentation et l'analyse de variables de pression comportant des capteurs de pression [pression artérielle, pression intracardiaque], des moyens de traitement des signaux délivrés par lesdits capteurs et des moyens d'affichage des variations desdits signaux, les moyens de traitement comportant une pluralité d'entrées pour recevoir des signaux analogiques provenant de différents capteurs, **caractérisé en ce que** chaque entrée est raccordée à un circuit d'échantillonnage fournissant un signal numérique exploité par un calculateur pour réaliser des traitements comprenant :
- le ré-échantillonnage des signaux visualisés ou enregistrés en blocs de N points, avec N étant puissance de deux,
- l'analyse de fréquence dudit signal échantillonné par rapport à une gamme de fréquences cibles, choisie parmi les ondes de type B, infra B et/ou ultra B, enregistrées dans une mémoire, pour la visualisation et l'enregistrement des variations temporelles des signaux correspondant aux ondes lentes,
- la détermination du décalage temporelle entre les ondes lentes des signaux correspondant à deux entrées distinctes.

2. Système pour la représentation et l'analyse de variables de pression selon la revendication 1 **caractérisé en ce que** les moyens d'analyse fréquentielle sont constitués par un calculateur appliquant une transformée de Fourier Rapide (FFT).

3. Système pour la représentation et l'analyse de variables de pression selon la revendication 1 **caractérisé en ce que** les moyens d'analyse fréquentielle sont constitués par un calculateur appliquant une analyse par ondelettes.

4. Système pour la représentation et l'analyse de variables de pression selon la revendication 1 **caractérisé en ce que** ladite gamme de fréquences cibles enregistrées comprend des fréquences comprises entre 8.10⁻³ Hertz et 50.10⁻³ Hertz.

5. Système pour la représentation et l'analyse de variables de pression selon l'une au moins des revendications précédentes **caractérisé en ce qu'**il comporte un circuit d'échantillonnage réalisant un échantillonnage de chacun des signaux d'entrée à une première fréquence, et un circuit de ré-échantillonnage à une fréquence d'échantillonnage inférieure à la première fréquence, pour l'enregistrement des signaux horodatés dans une mémoire (10).

6. Système pour la représentation et l'analyse de variables de pression selon l'une au moins des revendications précédentes **caractérisé en ce qu'**il comporte un module d'acquisition et de traitement des signaux local et au moins un poste de monitorage distant relié audit module d'acquisition locale par un réseau de télécommunication.

## Patentansprüche

1. System für die Darstellung und die Analyse von Druckvariablen, das Drucksensoren (arterieller Druck, intrakardialer Druck), Bearbeitungsmittel der Signale, die von den genannten Sensoren geliefert werden, und Mittel zur Anzeige der Schwankungen von den genannten Signalen umfasst, wobei die Bearbeitungsmittel eine Vielzahl an Eingängen umfasst, um analogische Signale zu empfangen, die von unterschiedlichen Sensoren stammen, **dadurch gekennzeichnet, dass** jeder Eingang an einen Bemusterungskreis angeschlossen ist, der ein numerisches Signal liefert, das von einem Kalkulator ausgewertet wird, um Bearbeitungen zu realisieren, die Folgendes umfassen:
- die neue Bemusterung der Signale, die in Blöcken von N Punkten angezeigt oder gespeichert werden, wobei N die Potenz zwei ist,
- die Analyse der Frequenz von dem genannten bemusterten Signal im Verhältnis zu einem Bereich von Zielfrequenzen, die zwischen den Wellen des Typs B, Infra-B und/oder Ultra-B ausgewählt werden, die in einem Speicher gespeichert sind, für die Anzeige und die Speicherung der zeitlichen Schwankungen der Signale, die den langsamen Wellen entsprechen,
- die Bestimmung der zeitlichen Verschiebung zwischen den langsamen Wellen der Signale, die zwei unterschiedlichen Eingängen entsprechen.

2. System für die Darstellung und die Analyse von Druckvariablen nach Anspruch 1, **dadurch gekennzeichnet, dass** die Mittel zur frequenziellen Analyse aus einem Kalkulator bestehen, der eine schnelle FourierTransformation (FFT) anwendet.

3. System für die Darstellung und die Analyse von Druckvariablen nach Anspruch 1, **dadurch gekennzeichnet, dass** die Mittel zur frequenziellen Analyse aus einem Kalkulator bestehen, der eine Analyse mit Wavelets anwendet.

4. System für die Darstellung und die Analyse von Druckvariablen nach Anspruch 1, **dadurch gekennzeichnet, dass** der genannte Bereich an Zielfrequenzen, die gespeichert sein, Frequenzen zwischen 8.10⁻³ Hertz und 50.10⁻³ Hertz umfasst.

5. System für die Darstellung und die Analyse von Druckvariablen nach mindestens einer der vorhergehenden Ansprüche, **dadurch gekennzeichnet, dass** es einen Bemusterungskreis umfasst, der eine Bemusterung von jedem der Eingangssignale an einer ersten Frequenz realisiert, und einen Neu-Bemusterungskreis mit einer Bemusterungsfrequenz, die unter der ersten Frequenz liegt, für die Speicherung der zeiterfassten Signale in einem Speicher (10).

6. System für die Darstellung und die Analyse von Druckvariablen nach mindestens einer der vorhergehenden Ansprüche, **dadurch gekennzeichnet, dass** es ein lokales Modul zur Erfassung und Bearbeitung der Signale und mindestens einen fernen Überwachungsposten umfasst, das über ein Telekommunikationsnetz mit dem genannten lokalen Modul zur Erfassung verbunden ist.

## Claims

1. System for representation and analysis of pressure variables comprising pressure sensors (blood pressure, intracardiac pressure), means of processing signals output by the said sensors and means of displaying variations of the said signals, the processing means comprising a plurality of inputs to receive the analogue signals output from the different sensors, **characterised in that** each input is connected to a sampling circuit outputting a digital signal used by a computer to perform processing including:
- re-sampling of displayed or recorded signals in blocks of N points, where N is the power of two,
- frequency analysis of the said sampled signal with respect to a range of target frequencies, chosen among type B, infra B and / or ultra B waves recorded in a memory to display and record variations with time of signals corresponding to slow waves,
- determination of the time offset between slow waves of signals, corresponding to two distinct inputs.

2. System for representation and analysis of pressure variables according to claim 1, **characterised in that** the frequency analysis means are composed of a computer applying a Fast Fourier Transform (FFT).

3. System for representation and analysis of pressure variables according to claim 1, **characterised in that** the frequency analysis means are composed of a computer applying an analysis by wavelets.

4. System for representation and analysis of pressure variables according to claim 1, **characterised in that** the said recorded target frequency range includes frequencies between 8 x 10⁻³ Hertz and 50 x 10⁻³ Hertz.

5. System for representation and analysis of pressure variables according to at least one of the previous claims, **characterised in that** it comprises a circuit that samples each of the input signals at a first frequency, and a re-sampling circuit working at a sampling frequency less than the first frequency, to record time dated signals in a memory (10).

6. System for representation and analysis of pressure variables according to at least one of the previous claims, **characterised in that** it comprises a local signal acquisition and processing module and at least one remote monitoring station connected to the said local acquisition module through a telecommunication network.
